# EUROPÄISCHE PATENTSCHRIFT

(11) **EP 2 136 125 B1**
(45) Veröffentlichungstag und Bekanntmachung des Hinweises auf die Patenterteilung: **11.08.2010**
(21) Anmeldenummer: 08011293.1
(22) Anmeldetag: 20.06.2008
(51) Int. Cl.: F21S 8/00, F21V 21/28, F21V 21/30, F21V 21/26, A61B 19/00

(54) **Operationsleuchte mit Aufhängevorrichtung**
Operation light with attachment device
Lampe chirurgicale dotée d'un dispositif de suspension

(43) Veröffentlichungstag der Anmeldung: 23.12.2009
(73) Patentinhaber: TRUMPF Medizin Systeme GmbH + Co. KG, 82178 Puchheim (DE)
(72) Erfinder: Marka, Rudolf, 85737 Ismaning (DE); Schmid, Michael, 82194 Gröbenzell (DE); Held, Fred, 22299 Hamburg (DE)
(74) Vertreter: Prüfer & Partner GbR European Patent Attorneys

(56) Entgegenhaltungen:
- EP-A- 0 121 414
- EP-A- 1 016 820
- EP-A- 1 087 176
- EP-A- 1 728 482
- DE-A1- 3 243 709
- DE-U1- 29 510 724

## Beschreibung

Die Erfindung betrifft eine Operationsleuchte mit einer Aufhängevorrichtung zum Aufhängen der Operationsleuchte an ein Tragsystem, womit die Operationsleuchte an einer Raumdecke, einer Wand oder einem fahrbaren Ständer befestigt wird.

Operationsleuchten werden an einer Raumdecke eines Operationssaals, einer Wand, oder als fahrbare Ausführungen an einem fahrbaren Ständer mit Hilfe eines so genannten Tragsystem befestigt. Das Tragsystem besteht üblicherweise aus einem ersten Ausleger, der um eine vertikale Achse schwenkbar ist und einem zweiten Ausleger, der horizontal schwenkbar und höhenverstellbar ist. Mit dem Tragsystem besteht die Möglichkeit, die Operationsleuchte an einer Stelle im Raum zu positionieren, von der aus es günstig ist, die Operationsstelle auszuleuchten.

Die Orientierung der Operationsleuchte und somit die Richtung des austretenden Lichts in den drei rotatorischen Freiheitsgraden wird durch eine gelenkige Aufhängevorrichtung ermöglicht.

Die Aufhängevorrichtung besteht üblicherweise in einer einfachen Ausführung aus einem Bügel mit einer annähernd kreisbogenartigen Form, der sich über einen Viertelkreis erstreckt (Viertel-Bügel) und der um eine vertikale erste Achse schwenkbar am äußeren Ende des zweiten Auslegers des Tragsystems an seinem oberen Ende befestigt ist. Am unteren Ende des Bügels befindet sich ebenfalls ein Drehgelenk, an dem der Leuchtenkörper der Operationsleuchte seitlich befestigt, um eine horizontale zweite Achse schwenkbar ist (halbkardanische Aufhängung). Der Nachteil dieser Ausführung ist eine aufwändigere Bedienung, da bestimmte Lichtrichtungen nicht direkt, sondern nur durch Verschwenken des Leuchtenkörpers um die zweite Achse eingestellt werden können, da der Leuchtenkörper nur um die eine horizontale Achse schwenkbar ist. Die Aufhängevorrichtung muss dann erst um die vertikale Achse gedreht werden, um bestimmte Lichtrichtungen einzustellen.

Um diesen Nachteil zu beheben und die Bedienung der Operationsleuchte komfortabler zu gestalten, wurde ein zweiter Bügel (Komfortbügel) eingeführt, der ebenfalls eine kreisbogenartige Form hat und sich über einen Viertelkreis erstreckt. Der Durchmesser des zweiten Bügels ist geringer als der Durchmesser des ersten Bügels, und die beiden Bügel sind konzentrisch um den virtuellen Mittelpunkt des ersten Bügels angeordnet. Der zweite Bügel ist an seinem ersten Ende mit dem ersten Bügel in der zweiten Drehachse drehbar verbunden und an seinem zweiten Ende ist der Leuchtenkörper, an einer dritten Drehachse schwenkbar, seitlich befestigt. Somit besitzt die Aufhängevorrichtung drei Drehachsen, wobei die erste und die zweite sowie die zweite und die dritte jeweils senkrecht zueinander stehen und es besteht die Möglichkeit den Leuchtenkörper nur durch eine Schwenkbewegung um die zweite und/oder dritte Achse in eine beliebige Richtung zu orientieren und somit die Richtung des Lichtaustritts wunschgemäß festzulegen (vollkardanische Aufhängung).

Die Europäische Patentanmeldung EP-A-1 087 176 offenbart diese Ausgestaltung der Aufhängevorrichtung mit zwei Bügeln und drei Drehachsen, deren Drehgelenk für die dritte Drehachse seitlich außerhalb des Leuchtenkörpers angeordnet ist.

Bei dieser Ausgestaltung entstehen allerdings die Nachteile, dass ein größerer Aufwand durch den zusätzlichen Bügel entsteht, Leuchtenkörper und Bügel mehr Bauraum einnehmen und die bewegte Masse vergrößert wird, was die Bewegungskraft erhöht. Außerdem muss berücksichtigt werden, dass sich die Bauhöhe der Aufhängevorrichtung durch den zusätzlichen Platzbedarf zum Durchschwenken des Viertel-Bügels unter dem senkrechten Drehgelenk um ca. 5 cm bis 8 cm vergrößert und somit die Durchgangshöhe unter dem Leuchtenköper reduziert ist. Dies kann, besonders in niedrigen Räumen und bei Operationsleuchtensystemen mit mehreren Operationsleuchten, die auf einer zentralen Achse in verschiedenen Höhen angebracht sind, zu einer Verletzungsgefahr durch Anstoßen des Kopfes des Operationspersonals führen.

Der Viertelbügel ist darüber hinaus hohen Drehmoment- bzw. Torsionsbelastungen durch das Leuchtenkörpergewicht ausgesetzt. Dadurch wird es schwierig, die beiden um 90° versetzten Achsen im Leuchtenkörperschwerpunkt zum Schnitt zu bringen (vertikaler Versatz). Ein weiteres Problem stellen Winkelabweichungen der Achsen von dem 90°-Winkel, der durch die Viertelkreisform des Viertelbügels gebildet wird, dar, weil dann ebenfalls nicht beide Drehachsen durch den Leuchtenköper-Schwerpunkt verlaufen (horizontaler Versatz). Somit ist es erforderlich, dass voreingestellte Leuchtenkörperpositionen durch stark eingestellt Bremsen gehalten werden müssen, was wiederum die Bedienkraft vergrößert.

Weitere bekannte Ausführungen sind im Wesentlichen konstruktive Abwandlungen des oben beschriebenen Systems aus Tragsystem und Aufhängevorrichtung. Beispielsweise ist eine Ausführung bekannt, in der ein halbkreisförmiger Bügel drehbar um die Längsachse des höhenverstellbaren Auslegers angebracht ist, der Bügel den Leuchtenkörper umgreift und der Leuchtenköper drehbar an den beiden Enden des Bügels gelagert ist.

Hierbei besteht der Nachteil, dass die zweite Drehachse schräg im Raum liegt, was ebenfalls zu einer schwierigeren Bedienung führt und dass der Leuchtenkörper nicht um seine Mittelachse gedreht werden kann, was insbesondere bei am Umfang angebrachten Bedienelementen zu Schwierigkeiten bei der Bedienung der Operationsleuchte führt. Die Bedienung ist hierbei vergleichbar zur halbkardanischen Aufhängung.

Es ist Aufgabe der Erfindung, diese Probleme zu lösen. Insbesondere ist es Aufgabe der Erfindung, eine Operationsleuchte mit einer Aufhängevorrichtung zur Verfügung zu stellen, die bei einer komfortablen Bedienung mit verringerter Bewegungskraft eine im Aufwand und im Gewicht reduzierte Aufhängevorrichtung zur Verfügung stellt, die eine reduzierte Bauhöhe aufweist.

Die Aufgabe wird mit den Merkmalen des Anspruchs 1 gelöst. Weiterbildungen der Erfindung sind Gegenstand der Unteransprüche.

Gemäß einem Aspekt der Erfindung wird eine Operationsleuchte offenbart, die über eine Aufhängevorrichtung mit mindestens zwei Drehgelenken mit einem Tragsystem verbunden ist, **dadurch gekennzeichnet, dass** die Aufhängevorrichtung ein erstes Drehgelenk mit einer ersten Drehachse senkrecht zur Mittelachse im zentralen Bereich des Leuchtenkörpers, in dem sich die erste Drehachse mit der Mittelachse schneidet, aufweist und der Leuchtenkörper um das erste Drehgelenk drehbar ist.

Die Anordnung der ersten Drehachse im zentralen Bereich des Leuchtenkörpers bietet den Vorteil, dass bei gleichem Bedienkomfort auf die Einführung eines zweiten Bügels verzichtet werden kann. Damit erspart man sich einen aufwändigen Bügel gegenüber dem erfindungsgemäßen einfachen Verbindungselement zwischen dem ersten und dem zweiten Drehgelenk. Da der Leuchtenköper ohne Drehbereichseingrenzung in zwei Drehachsen durchdrehbar sein soll, muss in einem konventionellen Fall auch für den Bügel, der den Leuchtenkörper teilweise umgibt, ein Freiraum zum Durchdrehen zur Verfügung gestellt werden, so dass sich im Grunde genommen der Durchmesser des Leuchtenkörpers vergrößert, was es erforderlich macht, die zweite Drehachse um ein bestimmtes Maß nach unten zu verlagern, was die Kopffreiheit des Operationspersonals verringert und somit die Unfallgefahr erhöht. Dies ist bei der Verwendung der Drehachse im zentralen Bereich nicht erforderlich.

Vorzugsweise weist die Aufhängevorrichtung der Operationsleuchte ein zweites Drehgelenk auf, dessen zweite Drehachse senkrecht zur ersten Drehachse ist. Dadurch ist eine einfache Bedienung gewährleistet, da sämtliche Schwenkungen um eine beliebige Achse in der horizontalen Ebene einfach durchgeführt werden können.

Vorteilhafterweise schneiden sich die erste und die zweite Drehachse in einem Schnittpunkt, der annähernd im Schwerpunkt des Leuchtenköpers liegt, so dass eine gleichmäßige Bedienkraft zum Verschwenken des Leuchtenkörpers ermöglicht wird. Bei einem größeren Abstand zwischen dem Schnittpunkt und dem Schwerpunkt liegt ein Hebelarm zwischen der im Schwerpunkt angreifenden Gewichtskraft und dem Schnittpunkt vor, und entweder muss der Schwerpunkt des Leuchtenkörpers gegen die wirkende Gewichtskraft nach oben geschwenkt werden, oder die wirkende Gewichtskraft unterstützt die Schwenkbewegung des Schwerpunkts des Leuchtenkörpers nach unten. Ansonsten besteht außerdem dann die Gefahr, dass sich der Leuchtenkörper selbsttätig bewegt und voreingestellte Leuchtenköperpositionen durch stark eingestellte Bremsen gehalten werden müssen, was wiederum die Bedienkraft vergrößert.

Zweckmäßig ist, dass die Operationsleuchte zwischen dem ersten und dem zweiten Gelenk der Aufhängevorrichtung ein Verbindungselement aufweist, das mindestens eine Länge hat, die gemeinsam mit den Abmessungen der Drehgelenke eine freie Durchdrehbarkeit des Leuchtenkörpers erlaubt, um die Durchdrehbarkeit des Leuchtenkörpers um die erste und die zweite Drehachse und somit das Einstellen jeder beliebigen Raumwinkelstellung zu ermöglichen. Vorteilhafterweise weist die Aufhängevorrichtung der Operationsleuchte ein drittes Drehgelenk mit einer dritten Drehachse auf, dessen dritte Drehachse vertikal orientiert ist und mit der zweiten Drehachse einen Winkel einschließt, der maximal 90° beträgt. Diese Drehachse ermöglicht es, den Leuchtenkörper um eine vertikale Achse zu drehen und somit neben jeder beliebigen Raumwinkelstellung, diese auch in jeder beliebigen Stellung der vertikalen dritten Drehachse der Operationsleuchte zu realisieren. Ein Winkel zwischen der dritten, vertikalen Drehachse und der zweiten Drehachse, der kleiner als 90° ist, bewirkt eine rotatorische Verlagerung des zweiten Drehgelenks um den Schnittpunkt der ersten und der zweiten Achse nach oben, was in diesem Bereich wiederum die Kopffreiheit für das Operationspersonal vergrößert.

Vorzugsweise weist die Aufhängevorrichtung der Operationsleuchte zwischen dem zweiten und dem dritten Drehgelenk ein zweites Verbindungselement auf, dessen Gestalt so bestimmt ist, dass die freie Durchdrehbarkeit des Leuchtenkörpers gegeben ist.

In einer vorteilhaften Ausführungsform ist das Verbindungselement bogenförmig oder aus mindestens zwei Abschnitten, die einen Winkel, der kleiner als 180° ist, einschließen, um den Materialeinsatz und damit die bewegten Massen zu reduzieren.

In einer weiteren Ausgestaltung der Erfindung weist die Aufhängevorrichtung der Operationsleuchte ein Kardangelenk mit der ersten und zweiten Drehachse auf, wobei das Kardangelenk im zentralen Bereich des Leuchtenkörpers angeordnet ist. Damit ist zwar die vollständig freie Positionierbarkeit nicht mehr uneingeschränkt gegeben, aber, da das Verbindungselement zwischen dem ersten und dem zweiten Drehgelenk entfällt, wird die Bedienbarkeit durch die Reduzierung der zu bewegenden Massen verbessert, und der Aufwand zur Herstellung weiter verringert.

Zweckmäßig ist, dass die Aufhängevorrichtung der Operationsleuchte mit dem Kardangelenk ein drittes Drehgelenk mit einer dritten Drehachse und ein Verbindungselement zwischen dem Kardangelenk und dem dritten Drehgelenk aufweist, das gerade ausgebildet ist, was die Bauhöhe und die zu bewegenden Massen weiter reduziert.

Vorteilhafterweise weist das erste Drehgelenk der Aufhängevorrichtung einen Verstellmechanismus auf, der in einer Richtung der, bzw. parallel zu der Mittelachse des Leuchtenkörpers verstellbar ist, um Toleranzen der Schwerpunktslage ausgleichen zu können und den Leuchtenkörper so einzustellen, dass der Schwerpunkt genau in dem Schnittpunkt der ersten und zweiten Drehachse liegt.

In einer weiteren bevorzugten Ausführungsform weist das erste Drehgelenk einen Verstellmechanismus auf, der in einer Richtung der, bzw. parallel zu der ersten Drehachse verstellbar ist, um Toleranzen der Schwerpunktslage ausgleichen zu können und den Leuchtenkörper so einzustellen, dass der Schwerpunkt genau in dem Schnittpunkt der ersten und zweiten Drehachse liegt.

Vorzugsweise weist das erste Drehgelenk einen Verstellmechanismus auf, der in einer Richtung senkrecht zu der ersten Drehachse und senkrecht zur Mittelachse des Leuchtenkörpers verstellbar ist, um Toleranzen der Schwerpunktslage ausgleichen zu können und den Leuchtenkörper so einzustellen, dass der Schwerpunkt genau in dem Schnittpunkt der ersten und zweiten Drehachse liegt. Die Erfindung wird anhand eines Ausführungsbeispiels unter Bezugnahme auf die beigefügten Zeichnungen erläutert.
- Fig. 1: ist eine Schrägansicht eines Ausführungsbeispiels der erfindungsgemäßen Operationsleuchte.
- Fig. 2: ist eine Schrägansicht eines Leuchtenkörpers und einer Aufhängevorrichtung des Ausführungsbeispiels mit einem Element eines dritten Drehgelenks.
- Fig. 3: zeigt eine Draufsicht auf den Leuchtenkörper und das erste Drehgelenk der Aufhängevorrichtung.
- Fig. 4: ist eine Schnittansicht des ersten Drehgelenks.

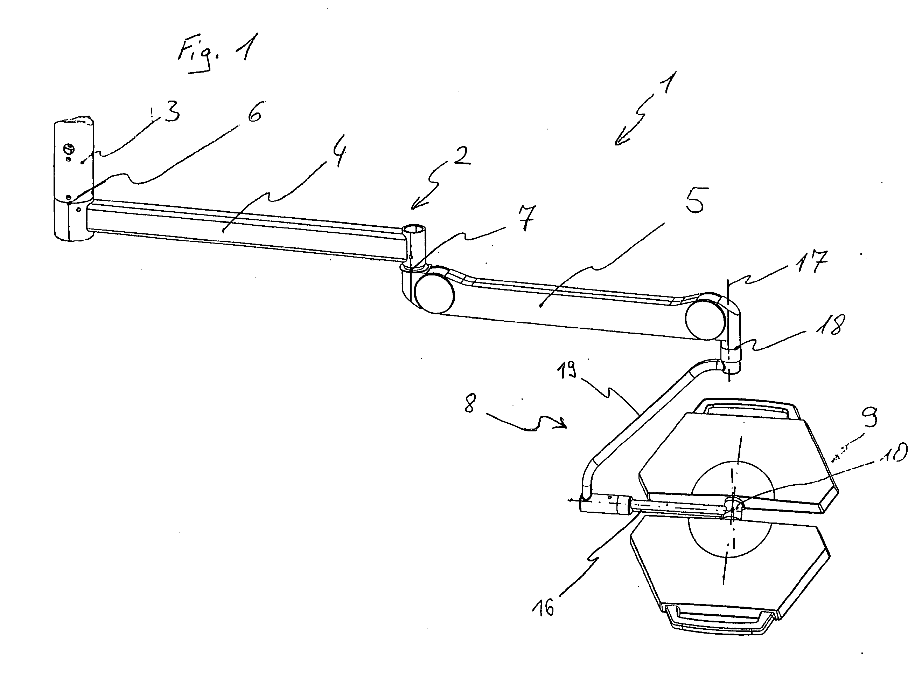

Fig. 1 zeigt eine Schrägansicht eines Ausführungsbeispiels der erfindungsgemäßen Operationsleuchte 1. Die Operationsleuchte 1 umfasst ein Tragsystem 2, das eine Zentralachse 3, einen ersten, um eine vertikale Achse schwenkbaren Ausleger 4, und einen zweiten, ebenfalls um die vertikale Achse schwenkbaren und zusätzlich höhenverstellbaren Ausleger 5 aufweist. Der erste Ausleger 4 ist über ein Drehgelenk 6 mit der Zentralachse 3 verbunden. Der zweite Ausleger 5 ist über ein Drehgelenk 7 mit dem ersten Ausleger 4 verbunden. Das Tragsystem wird an einer Raumdecke, einer Wand oder einem fahrbaren Ständer befestigt. Die Verbindung zu einer Aufhängevorrichtung 8 erfolgt über ein Drehgelenk 18 mit einer vertikalen Drehachse 17.

Das Tragsystem 2 ist mit einer nicht gezeigten elektrischen Versorgungs- und Steuerleitung zur Versorgung und Ansteuerung der Operationsleuchte, ausgehend von einem Versorgungssystem durch die Zentralachse 3, den ersten Ausleger 4 und den zweiten Ausleger 5 versehen. Die Drehgelenke 3, 7 sind ohne Anschläge ausgeführt, so dass die Ausleger 4, 5 in einem beliebigen Winkel in jede Schwenkrichtung verdreht werden können. Um dabei ein durchgängiges Kabel nicht zu beschädigen, sind in den Drehgelenken 3, 7 jeweils bekannte Schleifkontakte eingebaut, die mit den jeweiligen Kabelabschnitten verbunden sind.

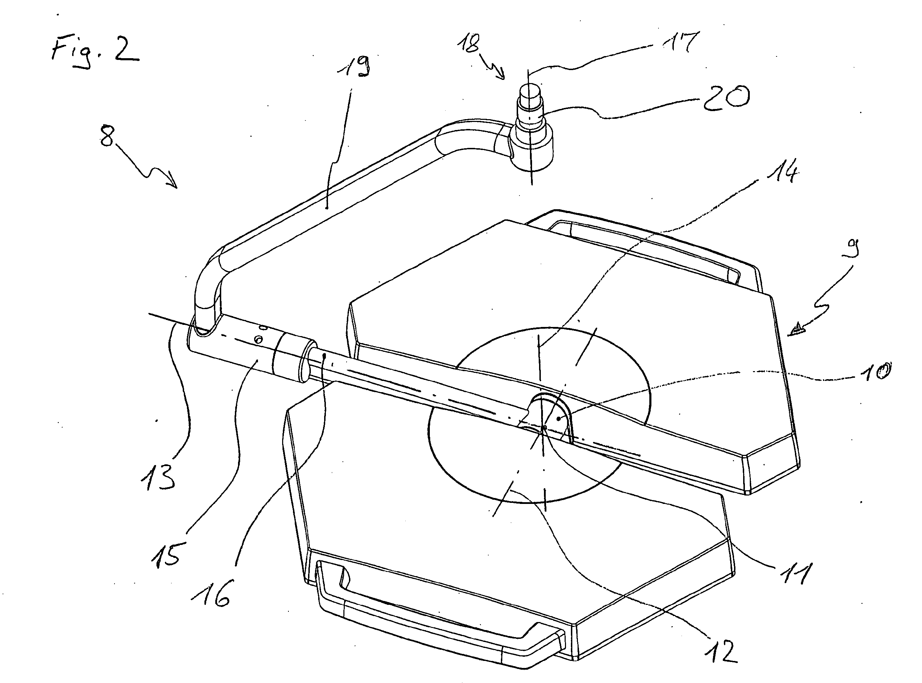

Fig. 2 zeigt eine Schrägansicht eines Leuchtenkörpers 9 und der Aufhängevorrichtung 8. Die Aufhängevorrichtung 8 besteht aus einem ersten Drehgelenk 10, in dem ein Schnittpunkt 11 einer ersten Drehachse 12 und einer zweiten Drehachse 13 eines zweiten Drehgelenks 13 liegt. Die erste Drehachse 12 und zweite Drehachse 13 stehen senkrecht aufeinander. Der Schnittpunkt 11 befindet sich in einem zentralen Bereich eines Leuchtenkörpers 9 und liegt in diesem Ausführungsbeispiel auf einer Mittelachse 14. Die erste Drehachse 12 stellt die Achse des ersten Drehgelenks 10 dar, die zweite Drehachse 13 stellt die Achse eines zweiten Drehgelenks 15 dar. In einem rechten Winkel zur zweiten Drehachse 13 liegt in diesem Ausführungsbeispiel die dritte, vertikale, Drehachse 17. In einem anderen Ausführungsbeispiel ist auch eine Ausführung möglich, bei der dieser Winkel kleiner als 90° ist, wodurch die Drehachse 13 nicht mehr horizontal im Raum liegt, sondern das Drehgelenk 15 in Bezug auf das erste Drehgelenk 10 und das dritte Drehgelenk 18 in einem Kreisbogen um die erste Drehachse 12 nach oben verlagert wird, was in diesem Bereich zu einer größeren Kopffreiheit für das Operationspersonal führt.

Das später näher beschriebene erste Drehgelenk 10 ist mit dem zweiten Drehgelenk 15 durch ein Verbindungselement 16 verbunden. Das Verbindungselement 16 besteht aus einem geraden, hohlen Stahlprofil und ist mit den Gelenken durch Schweißen verbunden. Der innere freie Querschnitt des Stahlprofils ist so gewählt, dass die erforderlichen elektrischen Leitungen durchgeführt werden können. Der Querschnitt des Stahlprofils ist so dimensioniert, dass das Flächenträgheitsmoment das durch die Gewichtskraft des Leuchtenkörpers 9 und die Bewegungskräfte verursachte Biegemoment mit den erforderlichen Sicherheitsfaktoren aufnehmen kann.

Das zweite Drehgelenk 15 ist mit dem dritten Drehgelenk 18 durch ein zweites Verbindungselement 19 verbunden. Dieses zweite Verbindungselement 19 besteht ebenfalls aus einem hohlen Stahlprofil. Für die Dimensionierung und Ausführung des Stahlprofils gelten die gleichen Anforderungen, wie für das Profil des ersten Verbindungselements 16. Die Gelenke sind mit dem Profil ebenfalls verschweißt.

Die Gestalt des Verbindungselements 19 ist so gewählt, dass die Ausführung raum- und materialsparend ist. Die vertikale Abmessung des Verbindungselements 19 ist, unter Berücksichtigung der Einklemmgefahr beim Durchdrehen des Leuchtenkörpers 9, möglichst gering, um den Leuchtenkörper 9 möglichst hoch anzubringen, und damit Kopffreiheit für das Operationspersonal zu gewinnen. Die horizontale Abmessung des Verbindungselements 19 ist so gewählt, dass unter Berücksichtigung der Abmessungen des ersten Drehgelenks 10, des zweiten Drehgelenks 15 und des Verbindungselements 16, der Schnittpunkt 11 auf der dritten Drehachse 17 liegt.

Die Verbindung der Aufhängevorrichtung 8 mit dem Tragsystem 2 wird über das dritte Drehgelenk 18 realisiert. Ein Anschlusszapfen 20 wird dabei in ein zylindrisches Gegenstück eingeführt und mit einer nicht gezeigten Scheibenfeder gesichert. Somit ist eine drehbare aber axial feste Verbindung realisiert. Als eine alternative Ausführungsform kann das Drehgelenk 18 durch eine starre Verbindung ersetzt werden.

Im zweiten und optional im dritten Drehgelenk 15, 18 werden die Bewegungskräfte durch den Einsatz von Wälzlagern reduziert, wobei aber, um ein selbsttätiges Verstellen des Leuchtenkörpers 9 zu vermeiden, die Gelenke mit einstellbaren Reibebremsen ausgerüstet sind.

Die Leitungsführung der elektrischen Leitungen ist in der Aufhängevorrichtung in den Verbindungselementen 16, 19 mit Hilfe von nicht gezeigten Kabeln und durch die Drehgelenke 15, 18 mit Hilfe von nicht gezeigten Schleifkontakten realisiert.

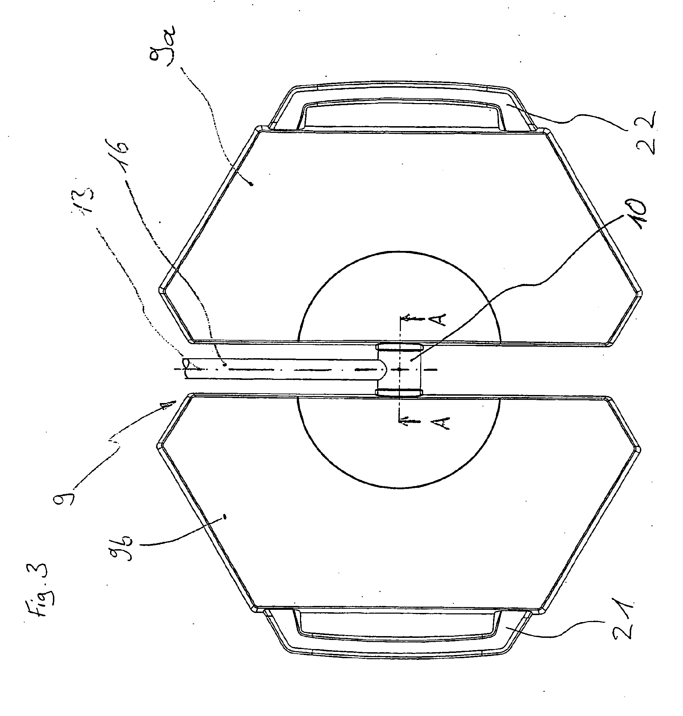

In Fig.3 ist eine Draufsicht auf den Leuchtenkörper 9 und das erste Drehgelenk 10 der Aufhängevorrichtung 8 gezeigt. Der Leuchtenkörper 9 besteht aus einer Hälfte 9a und einer Hälfte 9b, die durch einen nicht gezeigten Griff drehfest verbunden sind. Dadurch wird eine Drehbewegung der einen Hälfte auf die andere Hälfte übertragen und somit ist die Orientierung der beiden Hälften 9a und 9b immer gleich. Der Leuchtenkörper 9 wird über einen Griff 21, einen Griff 22 und über den nicht gezeigten Griff durch einen Bediener unsteril im Raum positioniert und so orientiert, dass das austretende Licht auf das Operationsfeld gerichtet ist. Zur Positionierung und Orientierung durch den sterilen Operateur ist ein weiterer, nicht gezeigter steriler Griff vorgesehen.

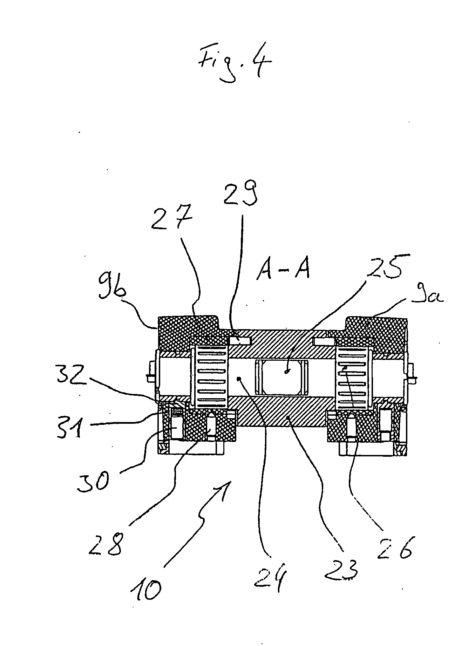

Fig. 4 ist eine Schnittansicht A-A des ersten Drehgelenks 10, deren Schnittverlauf in Fig. 3 gezeigt ist. Das Drehgelenk 10 besteht aus einer hohlen Welle 23, die durch Schweißen mit dem Verbindungselement 16 verbunden ist. Die Welle 23 ist mit einer Öffnung 25 versehen, die ungefähr die Abmessungen des inneren freien Querschnitts des Verbindungselements 16 hat und mit diesem zur Deckung gebracht ist.

Auf die Welle 23 sind als Wälzlager zwei Nadellager 26 aufgeschoben. Die Nadellager 26 sind an ihrem Außendurchmesser durch jeweils eine Buchse 27 umgeben, die an ihrem äußeren Umfang eine Abflachung mit einer kegelförmigen Senkung im Mittelpunkt der Abflachung besitzt, die in der Schnittebene sichtbar ist. Die Buchsen 27 und damit die äußeren Ringe der Nadellager 26 werden durch jeweils eine Schraube 28 mit kegelförmiger Spitze befestigt.

Ein Anschlag, der ein Durchdrehen des Leuchtenkörpers um die erste Drehachse 12 verhindert, wird durch jeweils einen Stift 29 gebildet, der in der Welle 24 eingepresst ist. Ein nicht gezeigtes Gegenstück ist jeweils in den Hälften 9a und 9b des Leuchtenkörpers 9 so befestigt, dass bei der Schwenkbewegung des Leuchtenkörpers 9 zwischen dem nicht gezeigten Griff und dem Verbindungselement 16 ein Freiraum mit ausreichender Größe bleibt, um ein Einklemmen der Hand zu vermeiden.

Radial zu der Welle 24 ist in beiden Hälften 9a und 9b des Leuchtenkörpers 9 (hier nur in der Hälfte 9b gezeigt) auf der Unterseite ein Bremssystem, bestehend aus einer Madenschraube 30, einem Tellerfedernpaket 31 und einem Bremsbelag 32, der kraftschlüssig, durch den Eingriff eines Stegs in eine umlaufende Nut der Welle 23 gegen Verdrehung gesichert, eine Bremskraft aufbringt.

Durch den inneren freien Querschnitt des Verbindungselements 16 und die Öffnung 25 sind die Kabel der Versorgungs- und Steuerleitungen für den Leuchtenkörper 9 in einer Bohrung 24 der Welle 23 verlegt und werden von dort in die Hälften 9a und 9b des Leuchtenkörpers 9 geführt. Die elektrische Verbindung der beiden Hälften 9a und 9b erfolgt ebenfalls über Kabel durch die Bohrung 24 der Welle 23. Schleifkontakte sind hier nicht erforderlich, da Anschläge vorhanden sind, die ein Durchdrehen des Leuchtenkörpers verhindern.

In den beiden Hälften 9a und 9b des Leuchtenkörpers 9 sind nicht gezeigte Leuchtmittel, die Licht nach unten in die Operationsstelle abgeben, und Steuerungs- und Versorgungsvorrichtungen für die Leuchtmittel angeordnet.

## Patentansprüche

1. Operationsleuchte, umfassend einen Leuchtenkörper (9) mit einer Mittelachse (14), der über eine Aufhängevorrichtung (8), die mindestens ein erstes Drehgelenk (10) mit einer ersten Drehachse (12) senkrecht zu der Mittelachse (14), und ein zweites Drehgelenk (15) aufweist, mit einem Tragsystem (2) verbunden ist, wobei der Leuchtenkörper (9) um das erste Drehgelenk (10) drehbar ist, **dadurch gekennzeichnet, dass** die Aufhängevorrichtung (8) das erste Drehgelenk (10) in einem zentralen Bereich des Leuchtenkörpers (9), in dem sich die erste Drehachse (12) mit der Mittelachse (14) schneidet, aufweist.

2. Operationsleuchte gemäß Anspruch 1, **dadurch gekennzeichnet, dass** die Aufhängevorrichtung (8) ein zweites Drehgelenk (15) mit einer zweiten Drehachse (13) aufweist, dessen zweite Drehachse (13) senkrecht zur ersten Drehachse (12) ist.

3. Operationsleuchte gemäß einem der vorangehenden Ansprüche, **dadurch gekennzeichnet, dass** sich die erste Drehachse (12) und die zweite Drehachse (13) im Schwerpunkt des Leuchtenkörpers (9) schneiden.

4. Operationsleuchte gemäß einem der Ansprüche 2 oder 3,
**dadurch gekennzeichnet, dass** die Aufhängevorrichtung (8) ein erstes Verbindungselement (16) zwischen dem ersten Drehgelenk (10) und dem zweiten Drehgelenk (15) aufweist, das eine Länge aufweist, so dass gemeinsam mit den Abmessungen der Drehgelenke (10, 15) eine freie Durchdrehbarkeit des Leuchtenkörpers (9) gegeben ist.

5. Operationsleuchte gemäß einem der Ansprüche 3 oder 4,
**dadurch gekennzeichnet, dass** die Aufhängevorrichtung (8) ein drittes Drehgelenk (18) mit einer dritten Drehachse (17) aufweist, dessen dritte Drehachse (17) vertikal orientiert ist und mit der zweiten Drehachse (13) einen Winkel einschließt, der maximal 90° beträgt.

6. Operationsleuchte gemäß Anspruch 5, **dadurch gekennzeichnet, dass** die Aufhängevorrichtung (8) ein zweites Verbindungselement (19) zwischen dem zweiten Drehgelenk (15) und dem dritten Drehgelenk (18) aufweist, dessen Gestalt so bestimmt ist, dass der Leuchtenkörper (9) durchdrehbar ist.

7. Operationsleuchte gemäß Anspruch 6, **dadurch gekennzeichnet, dass** das zweite Verbindungselement (19) bogenförmig ist.

8. Operationsleuchte gemäß Anspruch 6, **dadurch gekennzeichnet, dass** das zweite Verbindungselement (19) aus mindestens zwei Abschnitten besteht, die einen Winkel, der kleiner als 180° ist, einschließen.

9. Operationsleuchte gemäß Anspruch 2, **dadurch gekennzeichnet, dass** die Aufhängevorrichtung (8) ein drittes Drehgelenk (18) und ein Kardangelenk für die erste Drehachse (12) und zweite Drehachse (13), das im zentralen Bereich angeordnet ist, und ein Verbindungselement zwischen dem Kardangelenk und dem dritten Drehgelenk (18) aufweist.

10. Operationsleuchte gemäß Anspruch 9, **dadurch gekennzeichnet, dass** das Verbindungselement gerade ist.

11. Operationsleuchte gemäß einem der vorangehenden Ansprüche, **dadurch gekennzeichnet, dass** das erste Drehgelenk (10) einen Verstellmechanismus aufweist, der in einer Richtung in bzw. parallel zur Mittelachse (14) des Leuchtenkörpers (9) verstellbar ist.

12. Operationsleuchte gemäß einem der vorangehenden Ansprüche, **dadurch gekennzeichnet, dass** das erste Drehgelenk (10) einen Verstellmechanismus aufweist, der in einer Richtung in bzw. parallel zur ersten Drehachse (12) verstellbar ist.

13. Operationsleuchte gemäß einem der vorangehenden Ansprüche, **dadurch gekennzeichnet, dass** das erste Drehgelenk (10) einen Verstellmechanismus aufweist, der in einer Richtung senkrecht zur ersten Drehachse (12) und senkrecht zur Mittelachse (14) des Leuchtenkörpers (9) verstellbar ist.

## Claims

1. Operation light comprising
a lamp body (9) having a central axis (14), connected to a carrying system (2) by means of a suspension device (8) comprising at least a first pivot joint (10) having a first pivot axis (12) perpendicular to the central axis (14) and a second pivot joint (15), wherein the lamp body (9) is pivotable about the first pivot joint (10), **characterized in that**
the suspension device (8) comprises the first pivot joint (10) in a central area of the lamp body (9) in which the first pivot axis (12) intersects the central axis (14).

2. Operation light according to claim 1, **characterized in that** the suspension device (8) comprises a second pivot joint (15) with a second pivot axis (13), the second pivot axis (13) of which is perpendicular to the first pivot axis (12).

3. Operation light according to any of the preceding claims, **characterized in that** the first pivot axis (12) and the second pivot axis (13) intersect at a center of gravity of the lamp body (9).

4. Operation light according to any of claims 2 or 3, **characterized in that** the suspension device (8) comprises a first connection element (16) between the first pivot joint (10) and the second pivot joint (15), which has a length such that, together with the dimensions of the pivot joints (10, 15), the lamp body (9) can feely rotate.

5. Operation light according to any of claims 3 or 4, **characterized in that** the suspension device (8) comprises a third pivot joint (18) with a third pivot axis (17), the third pivot axis (17) of which is vertically orientated and forms a maximum angle of 90° with the second pivot axis (13).

6. Operation light according to claim 5, **characterized in that** the suspension device (8) comprises a second connection element (19) between the second pivot joint (15) and the third pivot joint (18), the shape of which is configured to allow the lamp body (9) to freely rotate.

7. Operation light according to claim 6, **characterized in that** the second connection element (19) is arc-shaped.

8. Operation light according to claim 6, **characterized in that** the second connection element (19) consists of at least two portions arranged at an angle of less than 180° relative to one another.

9. Operation light according to claim 2, **characterized in that** the suspension device (8) comprises a third pivot joint (18) and a cardan joint for the first pivot axis (12) and the second pivot axis (13), which is arranged in the central area, and **in that** a connection element is provided between the cardan joint and the third pivot joint (18).

10. Operation light according to claim 9, **characterized in that** the connection element has a linear shape.

11. Operation light according to any of the preceding claims, **characterized in that** the first pivot joint (10) comprises an adjusting mechanism which is adjustable in a direction along or rather parallel to the central axis (14) of the lamp body (9).

12. Operation light according to any of the preceding claims, **characterized in that** the first pivot joint (10) comprises an adjusting mechanism which is adjustable in a direction along or rather parallel to the first pivot axis (12).

13. Operation light according to any of the preceding claims, **characterized in that** the first pivot joint (10) comprises an adjusting mechanism which is adjustable in a direction which is perpendicular to the first pivot axis (12) and perpendicular to the central axis (14) of the lamp body (9).

## Revendications

1. Lampe chirurgicale, comprenant un corps de lampe (9), avec un axe central (14), relié, par l'intermédiaire d'un dispositif de suspension (8), présentant au moins une première articulation tournante (10) avec un premier axe de rotation (12) perpendiculaire à l'axe central (14), et une deuxième articulation tournante (15), à un système support (2), le corps de lampe (9) étant susceptible de tourner autour de la première articulation tournante (10), **caractérisé en ce que** le dispositif de suspension (8) présente la première articulation tournante (10) dans une zone centrale du corps de lampe (9), dans laquelle le premier axe de rotation (12) coupe l'axe central (14).

2. Lampe chirurgicale selon la revendication 1, **caractérisée en ce que** le dispositif de suspension (8) présente une deuxième articulation tournante (15) avec un deuxième axe de rotation (13), dont le deuxième axe de rotation (13) est perpendiculaire au premier axe de rotation (12).

3. Lampe chirurgicale selon l'une des revendications précédentes, **caractérisée en ce que** le premier axe de rotation (12) et le deuxième axe de rotation (13) se coupent au centre de gravité du corps de lampe (9).

4. Lampe chirurgicale selon l'une des revendications 2 ou 3, **caractérisée en ce que** le dispositif de suspension (8) présente, entre la première articulation tournante (10) et la deuxième articulation tournante (15), un premier élément de liaison (16) d'une longueur telle que, conjointement avec les dimensions des articulations tournantes (10, 15), une possibilité de tourner librement est fournie au corps de lampe (9).

5. Lampe chirurgicale selon l'une des revendications 3 ou 4, **caractérisée en ce que** le dispositif de suspension (8) présente une troisième articulation tournante (18) avec un troisième axe de rotation (17), dont le troisième axe de rotation (17) est orienté verticalement et fait, avec le deuxième axe de rotation (13), un angle d'une valeur maximale de 90°.

6. Lampe chirurgicale selon la revendication 5, **caractérisée en ce que** le dispositif de suspension (8) présente un deuxième élément de liaison (19), entre la deuxième articulation tournante (15) et la troisième articulation tournante (18), dont la configuration est déterminée de manière que le corps de lampe (9) puisse effectuer des rotations.

7. Lampe chirurgicale selon la revendication 6, **caractérisée en ce que** le deuxième élément de liaison (19) est en forme d'arc.

8. Lampe chirurgicale selon la revendication 6, **caractérisée en ce que** le deuxième élément de liaison (19) est composé d'au moins deux tronçons, faisant entre eux un angle inférieur à 180°.

9. Lampe chirurgicale selon la revendication 2, **caractérisée en ce que** le dispositif de suspension (8) présente une troisième articulation tournante (18) et une articulation à la cardan pour le premier axe de rotation (12) et le deuxième axe de rotation (13), disposé dans la zone centrale, et un élément de liaison entre l'articulation à la cardan et la troisième articulation tournante (18).

10. Lampe chirurgicale selon la revendication 9, **caractérisée en ce que** l'élément de liaison est rectiligne.

11. Lampe chirurgicale selon l'une des revendications précédentes, **caractérisée en ce que** la première articulation tournante (10) présente un mécanisme d'orientation, susceptible d'être orienté dans une direction située dans ou parallèlement à l'axe central (14) du corps de lampe (9).

12. Lampe chirurgicale selon l'une des revendications précédentes, **caractérisée en ce que** la première articulation tournante (10) présente un mécanisme d'orientation, susceptible d'être orienté dans une direction située dans ou parallèlement au premier axe de rotation (12).

13. Lampe chirurgicale selon l'une des revendications précédentes, **caractérisée en ce que** la première articulation tournante (10) présente un mécanisme d'orientation, susceptible d'être orienté dans une direction perpendiculaire à un premier axe de rotation (12) et perpendiculairement à l'axe central (14) du corps de lampe (9).
